(19) Europäisches Patentamt
European Patent Office
Office européen des brevets

(11) Veröffentlichungsnummer: **0 249 867 B1**

(12) **EUROPÄISCHE PATENTSCHRIFT**

(45) Veröffentlichungstag der Patentschrift: **02.01.92**

(51) Int. Cl.$^5$: **C07C 69/653**, C07C 67/14, C07C 51/347

(21) Anmeldenummer: **87108368.9**

(22) Anmeldetag: **10.06.87**

# (54) Substituierte Alpha-Fluoracrylsäurephenylester.

(30) Priorität: **14.06.86 DE 3620050**

(43) Veröffentlichungstag der Anmeldung:
**23.12.87 Patentblatt 87/52**

(45) Bekanntmachung des Hinweises auf die Patenterteilung:
**02.01.92 Patentblatt 92/01**

(84) Benannte Vertragsstaaten:
**AT BE CH DE ES FR GB GR IT LI LU NL SE**

(56) Entgegenhaltungen:
**EP-A- 0 128 517**
**EP-A- 0 136 668**
**EP-A- 0 160 379**
**DE-A- 2 950 491**
**US-A- 4 259 407**

(73) Patentinhaber: **HOECHST AKTIENGESELLSCHAFT**
**Postfach 80 03 20**
**W-6230 Frankfurt am Main 80(DE)**

(72) Erfinder: **Heumüller, Rudolf, Dr.**
**Strandpromenade 3**
**W-6054 Rodgau(DE)**
Erfinder: **Herbrechtsmeier, Peter, Dr.**
**Friedrich-Stolze-Strasse 10**
**W-6240 Königstein/Taunus(DE)**
Erfinder: **Siegemund, Günter, Dr.**
**Frankfurter Strasse 21**
**W-6238 Hofheim am Taunus(DE)**
Erfinder: **Groh, Werner, Dr.**
**Geisenheimer Strasse 93**
**W-6000 Frankfurt am Main(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung bezieht sich auf Ester der α-Fluoracrylsäure mit substituierten Phenolen, Verfahren zur Herstellung dieser Ester und deren Verwendung.

Ester der α-Fluoracrylsäure sind bereits bekannt. So wird der Phenylester der α-Fluoracrylsäure dadurch hergestellt, daß Monofluoressigsäureethylester in Gegenwart von Natriumethylat mit Ethyloxalat umgesetzt wird, das erhaltene Natrium-α-fluoracrylat mit Thionylchlorid in α-Fluoracryloylchlorid umgewandelt wird und letzteres dann mit Phenol verestert wird (deutsche Patentschrift 29 50 491 = US-Patentschrift 4 297 466). Hierbei ist nachteilig, daß der hochtoxische Monofluoressigsäureethylester eingesetzt werden muß. Der α-Fluoracrylsäurephenylester ist polymerisierbar und dient zur Herstellung von Polymeren, die bei Raumtemperatur transparente bzw. durchsichtige oder lichtdurchlässige und farblose Feststoffe darstellen.

Weitere Ester der α-Fluoracrylsäure, insbesondere Butyl-α-fluoracrylat, sind herstellbar durch saure Hydrolyse des jeweiligen α-Hydroxymethyl-α-fluormalonats und anschliessende Decarboxylierung des Hydrolyseproduktes unter gleichzeitiger Alkoholabspaltung (britische Patentschrift 1 115 287). Allerdings ist diese Methode nur am Beispiel des Butyl-α-fluoracrylats beschrieben; unter Lichteinfluß polymerisiert der Ester rasch.

Weiterhin sind Polymere von fluorierten Acrylsäureestern bekannt, die der Formel $RHC=CR\text{-}CO\text{-}OR_1$ entsprechen, wobei R ein Wasserstoffatom, eine Methylgruppe oder ein Halogenatom und $R_1$ ein fluorierter Alkyl- oder Arylrest ist (US-Patentschrift 2 877 207); der Fluoralkylrest der Alkoholkomponente ist insbesondere ein Rest, der am Kohlenstoffatom der 1-Position zwei Wasserstoffatome aufweist, und über geeignete Arylreste sind überhaupt keine Detailangaben gemacht.

Ferner ist bekannt, daß Polymere von α-Halogenacrylsäureestern mit halogenhaltigen Alkoholkomponenten zur Herstellung von strahlungsempfindlichen Schutzschichten dienen (US-Patentschrift 4 259 407). Als Ausgangsmaterial werden Monomere der Formel $H_2C = CX\text{-}COOR$ verwendet, in der X ein Fluor-, Chlor- oder Bromatom ist und R eine fluorierte Alkyl-, Aryl- oder Alkoxygruppe darstellt. Unter den Polymeren, die eine aromatische Alkoholkomponente enthalten, ist namentlich nur Poly(m-trifluormethylphenyl-α-fluoracrylat) erwähnt; über irgendwelche Eigenschaften dieses Polymers oder des entsprechenden Monomers sind jedoch keinerlei Angaben vorhanden.

Schließlich ist auch bekannt, daß Polymere von α-Fluoracrylsäureestern als Kernmaterial für optische Fasern geeignet sind (Europäische Anmeldungsveröffentlichung 0 128 517). Als einziger Ester mit einer aromatischen Alkoholkomponente ist der α-Fluoracrylsäurephenylester formelmäßig dargestellt, aber es fehlen jegliche Angaben über Herstellung und Eigenschaften dieses Esters.

Aufgabe der Erfindung ist die Bereitstellung von Estern der α-Fluoracrylsäure mit substituierten Phenolen, die in wirtschaftlicher Weise unter Verwendung möglichst wenig toxischer Ausgangsmaterialien herstellbar sind und zu Produkten mit hoher Lichtdurchlässigkeit polymerisierbar sind.

Die Erfindung betrifft α-Fluoracrylsäurephenylester der Formel (1)

(1) 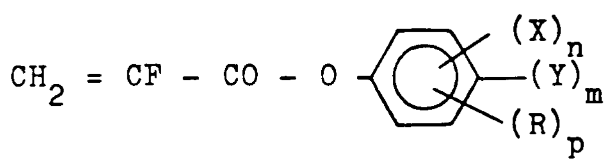

in der R eine Haloalkylgruppe mit 1 bis 8 Kohlenstoffatomen bedeutet und p Null, 1, 2 oder 3 ist, X ein Halogenatom bebedeutet und n Null oder eine ganze Zahl von 1 bis 5 ist (wobei X verschiedene Halogenatome bedeuten kann, wenn n größer als 1 ist), Y ein Wasserstoffatom, eine Cyanogruppe, den Rest X oder den Rest R bedeutet und m Null oder 1 ist, wobei die Summe n + m + p 2 bis 5 bedeutet.

Ferner betrifft die Erfindung ein Verfahren zur Herstellung eines substituierten α-Fluoracrylsäurephenylesters, das dadurch gekennzeichnet ist, daß in einem ersten Verfahrensschritt α-Fluormalonsäure-dimethylester mit Formaldehyd umgesetzt wird, dann in einem zweiten Verfahrensschritt der erhaltene hydroxymethylierte α-Fluormalonsäure-di-methylester hydrolysiert, decarboxyliert und dehydratisiert wird und anschließend in einem dritten Verfahrensschritt die erhaltene α-Fluoracrylsäure (gegebenenfalls in Form eines Säurehalogenids) mit einem Phenol der Formel (2)

(2)

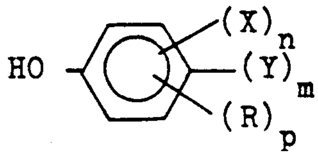

in der X, Y, R, n, m und p die bei Formel (1) angegebene Bedeutung haben (gegebenenfalls in Form eines Alkaliphenolats) verestert wird.

Schließlich betrifft die Erfindung auch die Verwendung von substituierten α-Fluoracrylsäurephenylestern der Formel (1) als Augangsmaterial zur Herstellung von fluorhaltigen Polymeren.

Das erfindungsgemäße Verfahren wird in drei Stufen durchgeführt: Zunächst wird α-Fluormalonsäure-dimethylester mit Formaldehyd zu α-Hydroxymethyl-α-fluormalonsäuredimethylester umgesetzt, dieser wird dann hydrolysiert und das Hydrolyseprodukt wird decarboxyliert und dehydratisiert, und schließlich wird die erhaltene α-Fluoracrylsäure mit einem substituierten Phenol verestert.

Im ersten Verfahrensschritt wird α-Fluormalonsäure-dimethylester einer Hydroxymethylierung mit Formaldehyd unterworfen. (α-Fluormalonsäure-dimethylester ist eine bekannte Verbindung; s. Journal of Fluorine Chemistry 25 (1984), 203 - 212.) Der Formaldehyd wird vorzugsweise in Form einer wäßrigen Lösung eingesetzt, die einen Formaldehydgehalt von 30 bis 40 Gewichtsprozent aufweist. Der Formaldehyd wird in einer Menge von 1 bis 10 mol, vorzugsweise 1,1 bis 3 mol, eingesetzt (bezogen auf 1 mol α-Fluormalonsäure-dimethylester). Statt Formaldehyd kann auch Paraformaldehyd, Hexamethylentetramin oder 1,3,5-Trioxan verwendet werden. Es ist vorteilhaft, die Umsetzung in Gegenwart eines basischen Katalysators durchzuführen, der dann in einer Menge von 2 bis 50, vorzugsweise von 5 bis 15 Molprozent (bezogen auf den α-Fluormalonsäure-dimethylester) verwendet wird. Als Katalysator dient insbesondere ein Alkalihydrogencarbonat, z.B. Kaliumhydrogencarbonat und Natriumhydrogencarbonat. Die Reaktion wird bei einer Temperatur von 5 bis 40 °C, vorzugsweise von 15 bis 30 °C durchgeführt. Der entstandene α-Hydroxymethyl-α-fluormalonsäure-dimethylester wird anschließend aus dem Reaktionsgemisch isoliert, vorzugsweise durch Aussalzen oder Extraktion mit Hilfe eines mit Wasser nicht mischbaren organischen Lösungsmittels. Als Lösungsmittel eignet sich vor allem ein aliphatischer Chlorkohlenwasserstoff mit 1 bis 4 Kohlenstoffatomen, z.B. Dichlormethan, Trichlormethan, Tetrachlormethan, 1,1-Dichlorethan, 1,2-Dichlorethan. Besonders vorteilhaft ist eine Kombination von Aussalzen und Extraktion; dabei wird das Reaktionsgemisch zunächst mit einer gesättigten Salzlösung (Ammoniumsulfat, Natriumchlorid) versetzt, und dieses Gemisch wird dann extrahiert. Durch Verdampfen des Lösungsmittels wird α-Hydroxymethyl-α-fluormalonsäuredimethylester als farbloser Feststoff gewonnen.

Im zweiten Verfahrensschritt wird der α-Hydroxymethyl-α-fluormalonsäure-dimethylester in einem wäßrigen sauren Medium hydrolysiert, und das Hydrolyseprodukt wird decarboxyliert und dehydratisiert. Die Reaktion wird bei einem pH-Wert von -1 bis 6, vorzugsweise 0 bis 2, durchgeführt; das saure Milieu wird mit Hilfe einer wäßrigen Säurelösung hergestellt, vorzugsweise einer verdünnten anorganischen Säure wie Salzsäure oder Schwefelsäure. Die Reaktionstemperatur liegt im Bereich von 90 bis 110 °C, vorzugsweise 95 bis 105 °C. Nach Beendigung der Gasentwicklung wird das Reaktionsgemisch bei einem Druck von 1013 bis 600 mbar destilliert, und das Destillat wird mit einem organischen Lösungsmittel extrahiert. Als Lösungsmittel wird hier ebenfalls ein mit Wasser nicht mischbares Lösungsmittel verwendet, vorzugsweise ein Ether wie Diethylether. Nach Verdampfen des Lösungsmittels wird α-Fluoracrylsäure als farbloser Feststoff erhalten. In einer bevorzugten Variante wird die α-Fluoracrylsäure als Ammoniumsalz isoliert. Dazu wird durch die nach der Extraktion erhaltene Lösung gasförmiges Ammoniak geleitet, und der Farblose kristalline Niederschlag wird dann vom Lösungsmittel befreit.

Im dritten Verfahrensschritt wird die α-Fluoracrylsäure mit einem substituierten Phenol verestert. Das Phenol wird in einer Menge von 0,5 bis 1,5 mol, vorzugsweise 0,8 bis 1,2 mol, eingesetzt (bezogen auf 1 mol α-Fluoracrylsäure). Gegebenenfalls - wenn die Säure in Form eines Halogenids eingesetzt wird - wird das Phenol in Form eines Alkaliphenolats, vorzugsweise Natriumphenolats oder Kaliumphenolats, eingesetzt. Für die Veresterung wird die α-Fluoracrylsäure als solche oder vorzugsweise in Form eines Säurehalogenids, insbesondere als α-Fluoracrylsäurechlorid, eingesetzt. Das Säurehalogenid wird mit Hilfe eines üblichen Halogenierungsmittels hergestellt, z.B. Oxalylchlorid, Phosphorpentachlorid, Phosphortrichlorid, Phosphoroxychlorid, Benzoylchlorid, Benzotrichlorid, Phosphortribromid, Schwefeltetrafluorid und insbesondere Thionylchlorid. Die Halogenierung mit Thionylchlorid erfolgt vorzugsweise in Gegenwart eines Katalysators wie Dimethylformamid. Die Umsetzung wird in einem aromatischen Kohlenwasserstoff, z.B. Toluol, Xylol und Trimethylbenzol, als Lösungsmittel durchgeführt, und die Reaktionstemperatur liegt im Bereich von 50 bis 100 °C, vorzugsweise 70 bis 90 °C.

Die Veresterung wird vorzugsweise in einem Lösungsmittel durchgeführt, und die Reaktionstemperatur

beträgt hier -10 bis 50 °C, vorzugsweise 0 bis 25 °C. Als Lösungsmittel dient ein polares organisches Lösungsmittel, insbesondere ein symmetrischer, asymmetrischer oder cyclischer Ether, z.B. Diethylether, Dipropylether, Diisopropylether, tert.-Butylmethylether, Tetrahydrofuran und Dioxan, ein aliphatischer Halogenkohlenwasserstoff, vorzugsweise Chlorkohlenwasserstoff, z.B. Dichlormethan, Trichlormethan, Tetrachlormethan, 1,1-Dichlorethan und 1,2-Dichlorethan, ein aromatischer Halogenkohlenwasserstoff, vorzugsweise Chlorkohlenwasserstoff, z.B. Chlorbenzol und 1,2- oder 1,3-Dichlorbenzol, oder ein aliphatisches oder aromatisches Nitril, z.B. Acetonitril und Benzonitril. Das Lösungsmittel kann auch ein Gemisch von mehreren polaren Lösungsmitteln sein. Es ist zweckmäßig, die Veresterung des Säurehalogenids mit dem Phenol in Gegenwart einer organischen Base durchzuführen, insbesondere eines Trialkylamins mit jeweils 1 bis 4 Kohlenstoffatomen in den Alkylgruppen. Die Base wird in einer Menge von 0,5 bis 2 mol, vorzugsweise 0,8 bis 1,2 mol, eingesetzt (bezogen auf 1 mol Säurehalogenid). Aus dem Reaktionsgemisch wird der erhaltene substituierte α-Fluoracrylsäure-phenylester durch Destillation, vorzugsweise bei einem Druck von 1013 bis 200 mbar, oder - nach destillativer Entfernung des Lösungsmittels - durch Heißextraktion des festen Rückstandes mit einem unpolaren Lösungsmittel, vorzugsweise einem aliphatischen Kohlenwasserstoff wie n-Hexan, und anschließende Kristallisation isoliert. Es ist zweckmäßig, die Destillation in Gegenwart eines üblichen Polymerisationsinhibitors, z.B. Hydrochinon oder Hydrochinonmonomethylether, durchzuführen; dieser wird in einer Menge von 100 bis 500 ppm (bezogen auf α-Fluoracrylsäurehalogenid) verwendet. Die Sumpftemperatur liegt im Bereich von 20 bis 100 °C, vorzugsweise 30 bis 85 °C. Zur weiteren Reinigung wird der Phenylester erneut destilliert, vorzugsweise bei vermindertem Druck, oder umkristallisiert.

Die erfindungsgemäßen substituierten α-Fluoracrylsäurephenylester tragen 2 bis 5 Substituenten am Phenylrest; bevorzugt sind Ester, die als Substituenten oder im Substituenten zwei oder mehrere Fluoratome enthalten.

Zu den erfindungsgemäßen Estern gehören beispielsweise die folgenden Verbindungen:
2,3- oder 2,4- oder 2,5- oder 2,6- oder 3,4- oder 3,5-Difluorphenyl-αfluoracrylat, 2,3- oder 2,4- oder 2,5- oder 2,6- oder 3,4- oder 3,5-Dichlorphenyl-α-fluoracrylat, 2- oder 3-Chlor-4-fluorphenyl-α-fluoracrylat, 2-Brom-4-chlorphenyl-α-fluoracrylat, 4-Brom-2-chlorphenyl-α-fluoracrylat, 2,6-Dibrom-4-fluorphenyl-α-fluoracrylat, 4-Brom-2,6-dichlorphenyl-α-fluoracrylat, 2,4-Dichlor-6-jodphenyl-α-fluoracrylat, 2,3,4- oder 2,3,5-oder 2,3,6- oder 2,4,5- oder 2,4,6- oder 3,4,5-Trichlorphenyl-α-fluoracrylat, 2,4,6-Tribromphenyl-α-fluoracrylat, 2,4,6-Trijodphenyl-α-fluoracrylat, 2,3,5,6-Tetrafluorphenyl-α-fluoracrylat, 2,3,4,5-Tetrachlorphenyl-α-fluoracrylat, 2,3,5,6-Tetrachlorphenyl-α-fluoracrylat, Pentafluorphenyl-α-fluoracrylat, Pentachlorphenyl-α-fluoracrylat, Pentabromphenyl-α-fluoracrylat, 2,6-Dijod-4-trifluormethylphenyl-α-fluoracrylat, 2,6- oder 3,5-Bis-(trifluormethyl)-phenyl-α-fluoracrylat, 2,6-Dibrom-4-cyanophenyl-α-fluoracrylat, 2,6-Dichlor-4-cyanophenyl-α-fluoracrylat, 2,6-Difluor-4-cyanophenyl-α-fluoracrylat, 4-Cyano-2,3,5,6-tetrafluorphenyl-α-fluoracrylat, 4-Chlor-2,5 (oder 3,5)-difluorphenyl-α-fluoracrylat, 2-Chlor-4, 5-oder 4,6)-difluorphenyl-α-fluoracrylat, 2,5- oder 2,6- oder 3,5-Dichlor-4-fluorphenyl-α-fluoracrylat, 2,6- oder 3,4-Dibrom-3,4 (oder 2,6)-dichlorphenyl-α-fluoracrylat, 2,4,6-Trichlor-3-fluorphenyl-α-fluoracrylat, 2,4- oder 2,6-Dichlor-3-trifluormethylphenyl-α-fluoracrylat, 2,6-Dibrom-4 (oder 5) -trifluormethylphenyl-α-fluoracrylat, 2,4-Dibrom-5 (oder 6)-trifluormethylphenyl-α-fluoracrylat, 2- oder 4-Brom-3 (oder 5)-trifluormethylphenyl-α-fluoracrylat, 2,3,6-Tribrom,4,5-bisbrommethylphenyl-α-fluoracrylat, 2,4,5-Tribrom-3,6-bis(brommethyl)phenyl-α-fluoracrylat, 2,4,6-Trichlor-3,5-bis(chlormethyl)-phenyl)-α-fluoracrylat, 3,4,5-Trichlor-2,6-bis(chlormethyl)phenyl-α-fluoracrylat, 2,4,6-Trichlor-3-chlormethylphenyl-α-fluoracrylat, 2,4-Dibrom-6-dibrommethylphenyl-α-fluoracrylat, 2,6-Dibrom-4-dibrommethylphenyl-α-fluoracrylat, 2,3,5,6-Tetrachlor-4-chlormethylphenyl-α-fluoracrylat, 2- oder 3-Fluor-4-methylphenyl-α-fluoracrylat, 3- oder 4-oder 5-Fluor-2-methylphenyl-α-fluoracrylat; 2-Fluor-6-methylphenyl-α-fluoracrylat, 4-Fluor-3-methylphenyl-α-fluoracrylat, 2,4,6-Trichlor-3,5-dimethylphenyl-α-fluoracrylat, 2,6-Dichlor-3,5-dimethylphenyl-α-fluoracrylat, 2,6-Dichlor-4-methylphenyl-α-fluoracrylat, 2,4,6-Tribrom-3,5-dimethylphenyl-α-fluoracrylat, 2,4,6-Tribrom-3-methylphenyl-α-fluoracrylat, 2,3,5,6-Tetrafluor-4-cyanophenyl-α-fluoracrylat 2,3- oder 2,4 oder 2,5- oder 2,6- oder 3,4- oder 3,5-Dimethylphenyl-α-fluoracrylat, 2,4,6-Trimethylphenyl-α-fluoracrylat, 2,3,5,6-Tetramethylphenyl-α-fluoracrylat, 2- oder 3- oder 4-Isopropylphenyl-α-fluoracrylat, 4-Isopropyl-3,5-dimethylphenyl-α-fluoracrylat und 2,3- oder 2,5- oder 2,6- oder 3,4- oder 3,5-Diisopropylphenyl-α-fluoracrylat.

Die substituierten Phenylester der α-Fluoracrylsäure sind bei Raumtemperatur farblose Flüssigkeiten oder farblose Feststoffe. Sie sind leicht polymerisierbar und eignen sich zur Herstellung von glasklaren, lichtdurchlässigen und farblosen Polymeren.

Die folgenden Beispiele dienen zur näheren Erläuterung der Erfindung. Prozentangaben beziehen sich jeweils auf das Gewicht.

Beispiel 1

a) In einem 4-Liter-Glaskolben wurde 48 g (0.48 mol) Kaliumhydrogencarbonat in 535 g (6,59 mol) wäßriger Formaldehydlösung (37gewichtsprozentig) gelöst. Zu dieser Lösung wurde 841 g (5,6 mol) α-Fluormalonsäure-dimethylesterunter Rühren innerhalb von 3 1/2 Stunden zugetropft; dabei wurde die Temperatur im Bereich von 20 bis 25° C gehalten. Während einer Nachrührzeit von 2 Stunden bei derselben Temperatur fiel α-Hydroxymethyl-α-fluormalonsäuredimethylester als farbloser Feststoff aus. Das Reaktionsgemisch wurde anschließend mit 2500 g einer wäßrigen gesättigten Ammoniumsulfatlösung versetzt und dann mit Dichlormethan extrahiert. Die Extraktionslösung wurde mit wasserfreiem Natriumsulfat getrocknet. Nach Abdestillieren des Dichlormethans (Badtemperatur 40° C, 25 mbar) wurden 906 g (90 Prozent der Theorie) α-Hydroxymethyl-α-fluormalonsäure-dimethylester erhalten.

b) In einem 2-Liter-Glaskolben, der mit Thermometer und Rührer und über eine Vigreux-Kolonne mit einem Destillationsaufsatz versehen war, wurde 175 g (0,97 mol) α-Hydroxymethyl-α-fluormalonsäure-dimethylester, 750 ml Wasser und 750 ml Salzsäure (36gewichtsprozentig) 2 1/2 Stunden lang zum Sieden erhitzt. Dabei betrug die Temperatur des Reaktionsgemisches 103° C. Anschließend wurde das Reaktionsgemisch destilliert. Das Destillat wurde mit 1 g Hydrochinonmonomethylether versetzt und mit Diethylether extrahiert, und die Extraktionslösung wurde mit wasserfreiem Natriumsulfat getrocknet. In die Lösung wurde dann bei Raumtemperatur 17 g (1 mol) gasförmiges Ammoniak eingeleitet. Der dadurch erhaltene farblose Niederschlag wurde abfiltriert, mit Diethylether gewaschen und bei Raumtemperatur und unter vermindertem Druck getrocknet. Es wurden 70,8 g (68 Prozent der Theorie) Ammonium-α-fluoracrylat erhalten.

c) In einem 1-Liter-Glaskolben wurden 100 g (0,934 mol) Ammonium-α-fluoracrylat in einem Gemisch aus 600 g Mesitylen und 15 ml Dimethylformamid dispergiert und innerhalb einer Stunde mit 119 g (1,0 mol) Thionylchlorid versetzt. Das erhaltene Gemisch wurde auf eine Temperatur von 80° C erhitzt und 2 Stunden lang unter Rühren bei dieser Temperatur gehalten. Die nach Erkalten auf Raumtemperatur erhaltene Flüssigkeit wurde bei vermindertem Druck destilliert, und die bis 100° C/160 mbar erhaltene Fraktion wurde nochmals bei Normaldruck destilliert. Es wurden 67 g (66 Prozent der Theorie) α-Fluoracrylsäurechlorid mit einem Siedepunkt von 65 bis 67° C erhalten.

d) Zu einer Lösung von 54,5 g (0,502 mol) α-Fluoracrylsäurechlorid in 100 ml trockenem Acetonitril wurde bei einer Temperatur von 25° C unter Rühren eine Lösung von 111 g (0,5 mol) Kaliumpentafluorphenolat in 250 ml trockenem Acetonitril innerhalb von 1 Stunde getropft, und das Reaktionsgemisch wurde dann weitere 2 Stunden lang bei derselben Temperatur gerührt. Der entstandene Feststoff wurde abfiltriert und mit 100 ml trockenem Acetonitril gewaschen. Das Gemisch aus Filtrat und Waschlösung wurde nach Zugabe von 0,01 g Hydrochinonmonomethylether destilliert. Es wurde 107,1 g (84 Prozent der Theorie) α-Fluoracrylsäurepentafluorphenylester mit einem Siedepunkt von 41 bis 42° C (bei 2 mbar) erhalten.

Beispiel 2

13,9 g (0,128 mol) α-Fluoracrylsäurechlorid (erhalten nach Beispiel 1c) wurden bei einer Temperatur von 25° C unter Rühren innerhalb von 30 min zu einer Lösung von 30 g (0,0985 mol) Kaliumpentachlorphenolat in 400 ml trockenem Acetonitril getropft, und das Reaktionsgemisch wurde dann noch 90 min lang bei derselben Temperatur gerührt. Der entstandene Feststoff wurde abfiltriert und mit 20 ml trockenem Acetonitril gewaschen. Aus dem Gemisch aus Filtrat und Waschlösung wurde die flüchtigen Bestandteile nach Zugabe von 0,01 g Hydrochinonmonomethylether abdestilliert, und der Rückstand wurde aus siedendem n-Hexan umkristallisiert. Es wurden 17 g (51 Prozent der Theorie) α-Fluoracrylsäurepentachlorphenylester mit einem Schmelzpunkt von 90 bis 92° C erhalten.

Beispiel 3

49 g (0,181 mol) 2,6-Dibrom-4-fluorphenol wurden in 150 ml trockenem Dichlormethan gelöst, und die Lösung wurde mit 18,3 g (0,181 mol) Triethylamin versetzt; anschließend wurden bei einer Temperatur von 5° C unter Rühren innerhalb von 30 min 20 g (0,184 mol) α-Fluoracrylsäurechlorid (erhalten nach Beispiel 1c) zugetropft, und das Reaktionsgemisch wurde noch 30 min bei derselben Temperatur gerührt. Der entstandene Feststoff wurde abfiltriert und mit 30 ml Dichlormethan gewaschen. Das Gemisch aus Filtrat und Waschlösung wurde nach Zugabe von 0,01 g Hydrochinonmonomethylether destilliert. Es wurden 46 g (74 Prozent der Theorie) α-Fluoracrylsäure-2,6-dibrom-4-fluorphenylester mit einem Siedepunkt von 86 bis 88° C (bei 0,4 mbar) erhalten.

Beispiel 4

2,2 g (0,02 mol) α-Fluoracrylsäurechlorid (erhalten nach Beispiel 1c) wurden bei einer Temperatur von 5° C unter Rühren innerhalb von 5 min zu einer Lösung von 7,8 g (0,019 mol) 2,6-Dijod-4-trifluormethylphenol und 2 g (0,02 mol) Triethylamin in 80 ml trockenem Dichlormethan getropft, und das Reaktionsgemisch wurde dann noch 30 min lang bei derselben Temperatur gerührt. Der entstandene Feststoff wurde ab filtriert und mit 10 ml trockenem Dichlormethan gewaschen. Aus dem Gemisch aus Filtrat und Waschlösung wurden die flüchtigen Bestandteile nach Zugabe von 0,001 g Hydrochinonmonomethylether abdestilliert, und der Rückstand wurde aus siedendem n-Hexan umkristallisiert. Es wurden 7 g (76 Prozent der Theorie) α-Fluoracrylsäure-2,6-dijod-4-trifluormethylphenylester mit einem Schmelzpunkt von 93 bis 95° C erhalten.

Beispiel 5

16,2 g (0,1 mol) 4-Trifluormethylphenol wurden in 80 ml trockenem Diethylether gelöst, und die Lösung wurde portionsweise mit 10,1 g (0,1 mol) Triethylamin versetzt; anschließend wurden bei einer Temperatur von 10° C unter Rühren innerhalb von 20 min 12 g (0,11 mol) α-Fluoracrylsäurechlorid (erhalten nach Beispiel 1c) zugetropft, und das Reaktionsgemisch wurde noch 60 min bei derselben Temperatur gerührt. Der entstandene Feststoff wurde abfiltriert und mit 20 ml Diethylether gewaschen. Das Gemisch aus Filtrat und Waschlösung wurde nach Zugabe von 0,005 g Hydrochinonmonomethylether destilliert. Es wurden 19,5 g (83 Prozent der Theorie) α-Fluoracrylsäure-(4-trifluormethyl)-phenylester mit einem Siedepunkt von 69 bis 70° C (bei 3 mbar) erhalten.

Beispiel 6

27,5 g (0,169 mol) 3-Trifluormethylphenol wurden in 100 ml trockenem Dichlormethan gelöst, und die Lösung wurde portionsweise mit 17,1 g (0,169 mol) Triethylamin versetzt; anschließend wurden bei einer Temperatur von 5° C unter Rühren innerhalb von 20 min 20 g (0,184 mol) α-Fluoracrylsäurechlorid(erhalten nach Beispiel 1c) zugetropft, und das Reaktionsgemisch wurde noch 60 min bei derselben Temperatur gerührt. Der entstandene Feststoff wurde abfiltriert und mit 30 ml Dichlormethan gewaschen. Das Gemisch aus Filtrat und Waschlösung wurde nach Zugabe von 0,01 g Hydrochinonmonomethylether destilliert. Es wurden 27 g (68 Prozent der Theorie) α-Fluoracrylsäure-(3-trifluormethyl)-phenylester mit einem Siedepunkt von 78 bis 79° C (bei 4 mbar) erhalten.

Beispiel 7

26,2 g (0,1 mol) 4-Perfluorisopropylphenol wurden in 100 ml trockenem Diethylether gelöst, und die Lösung wurde mit 10,1 g (0,1 mol) Triethylamin versetzt; anschließend wurden bei einer Temperatur von 10° C unter Rühren innerhalb von 15 min 12 g (0,11 mol) α-Fluoracrylsäurechlorid (erhalten nach Beispiel 1c) zugetropft, und das Reaktionsgemisch wurde noch 30 min bei derselben Temperatur gerührt. Der entstandene Feststoff wurde abfiltriert und mit 20 ml Diethylether gewaschen. Das Gemisch aus Filtrat und Waschlösung wurde nach Zugabe von 0,005 g Hydrochinonmonomethylether destilliert. Es wurde 22,9 g (69 Prozent der Theorie) α-Fluoracrylsäure-(4-perfluorisopropyl)-phenylester mit einem Siedepunkt von 65 bis 66° C (bei 1 mbar) erhalten.

Beispiel 8

10 g (0,038 mol) 2-Perfluorisopropylphenol wurden in 30 ml trockenem Diethylether gelöst, und die Lösung wurde mit 3,9 g (0,038 mol) Triethylamin versetzt; anschließend wurden bei einer Temperatur von 10° C unter Rühren innerhalb von 12 min 4,3 g (0,039 mol) α-Fluoracrylsäurechlorid (erhalten nach Beispiel 1c) zugetropft, und das Reaktionsgemisch wurde noch 30 min bei derselben Temperatur gerührt. Der entstandene Feststoff wurde abfiltriert und mit 10 ml Diethylether gewaschen. Das Gemisch aus Filtrat und Waschlösung wurde nach Zugabe von 0,002 g Hydrochinonmonomethylether destilliert. Es wurden 7 g (55 Prozent der Theorie) α-Fluoracrylsäure-(2-perfluorisopropyl)-phenylester mit einem Siedepunkt von 85° C (bei 1 mbar) erhalten.

Beispiel 9

10 g (0,092 mol) α-Fluoracrylsäurechlorid (erhalten nach Beispiel 1c) wurden bei einer Temperatur von 5° C unter Rühren innerhalb von 20 min zu einer Lösung von 10,7 g (0,09 mol) 4-Cyanophenol und 9,1 g (0,09 mol) Triethylamin in 150 ml trockenem Dichlormethan getropft, und das Reaktionsgemisch wurde

dann noch 30 min lang bei derselben Temperatur gerührt. Der entstandene Feststoff wurde abfiltriert und mit 20 ml trockenem Dichlormethan gewaschen. Aus dem Gemisch aus Filtrat und Waschlösung wurden nach Zugabe von 0,005 g Hydrochinonmonomethylether die flüchtigen Bestandteile abdestilliert, und der Rückstand wurde aus siedendem n-Hexan umkristallisiert. Es wurden 11,2 g (65 Prozent der Theorie) α-Fluoracrylsäure-4-cyanophenylester mit einem Schmelzpunkt von 78 bis 79° C erhlaten.

Beispiel 10

10 g (0,092 mol) α-Fluoracrylsäurechlorid (erhalten nach Beispiel 1c) wurden bei einer Temperatur von 5° C unter Rühren innerhalb von 20 min zu einer Lösung von 16,7 g (0,089 mol) 3,5-Dichlor-4-hydroxybenzonitril und 9 g (0,089 mol) Triethylamin in 200 ml trockenem Dichormethan getropft, und das Reaktionsgemisch wurde dann noch 30 min lang bei derselben Temperatur gerührt. Der entstandene Feststoff wurde abfiltriert und mit 20 ml trockenem Dichlormethan gewaschen. Aus dem Gemisch aus Filtrat und Waschlösung wurden nach Zugabe von 0,005 g Hydrochinonmonomethylether die flüchtigen Bestandteile abdestilliert, und der Rückstand wurde aus siedendem n-Heptan umkristallisiert. Es wurden 18,7 g (81 Prozent der Theorie) α-Fluoracrylsäure-2,6-dichlor-4-cyanophenylester mit einem Schmelzpunkt von 95 bis 99° C erhalten.

Beispiel 11

10 g (0,092 mol) α-Fluoracrylsäurechorid (erhalten nach Beispiel 1c) wurden bei einer Temperatur von 5° C unter Rühren innerhalb von 5 min zu einer Lösung von 24,8 g (0,09 mol) 3,5-Dibrom-4-hydroxybenzonitril und 9,1 g (0,09 mol) Triethylamin in 200 ml Trockenem Dichlormethan getropft, und das Reaktionsgemisch wurde dann noch 30 min lang bei derselben Temperatur gerührt. Der entstandene Feststoff wurde abfiltriert und mit 20 ml trockenem Dichlormethan gewaschen. Aus dem Gemisch aus Filtrat und Waschlösung wurden nach Zugabe von 0,005 g Hydrochinonmonomethylether die flüchtigen Bestandteile abdestilliert, und der Rückstand wurde aus siedendem n-Heptan umkristalliesiert. Es wurden 17,5 g (55 Prozent der Theorie) α-Fluoracrylsäure-2,6-dibrom-4cyanophenylester mit einem Schmelzpunkt von 135 bis 138° C erhalten.

## Patentansprüche

**Patentansprüche für folgende Vertragsstaaten : BE, CH, DE, FR, GB, IT, LI, LU, NL, SE**

**1.** α-Fluoracrylsäurephenylester der Formel (1)

(1) 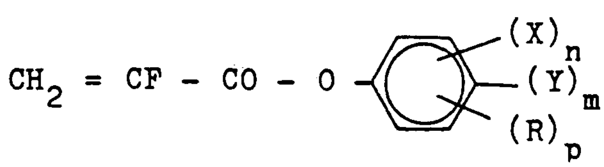

in der R eine Haloalkylgruppe mit 1 bis 8 Kohlenstoffatomen bedeutet und p Null, 1, 2 oder 3 ist, X ein Halogenatom bedeutet und n Null oder eine ganze Zahl von 1 bis 5 ist (wobei X verschiedene Halogenatome bedeuten kann, wenn n größer als 1 ist), Y ein Wasserstoffatom, eine Cyanogruppe, den Rest X oder den Rest R bedeutet und m Null oder 1 ist, wobei die Summe n + m + p 2 bis 5 beträgt.

**2.** Verfahren zur Herstellung eines α-Fluoracrylsäurephenylesters, dadurch gekennzeichnet, daß in einem ersten Verfahrensschritt α-Fluormalonsäure-dimethylester mit Formaldehyd umgesetzt wird, dann in einem zweiten Verfahrensschritt der erhaltene hydroxymethylierte α-Fluormalonsäure-dimethylester hydrolysiert, decarboxyliert und dehydratisiert wird und anschließend in einem dritten Verfahrensschritt die erhaltene α-Fluoracrylsäure (gegebenenfalls in Form eines Säurehalogenids) mit einem Phenol der Formel (2)

(2) 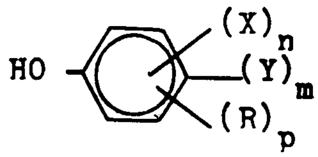

in der X, Y, R, n, m und p die bei Formel (1) angegebene Bedeutung haben (gegebenenfalls in Form eines Alkaliphenolats) verestert wird.

**3.** Verfahren nach Anspruch 2, dadurch gekennzeichnet, daß der erste Verfahrensschritt bei einer Temperatur von 5 bis 40° C, der zweite Verfahrensschritt bei einer Temperatur von 90 bis 110° C und der dritte Verfahrensschritt bei einer Temperatur von -10 bis 50° C durchgeführt werden.

**4.** Verwendung des α-Fluoracrylsäurephenylesters der Formel (1) als Ausgangsmaterial zur Herstellung eines fluorhaltigen Polymers.

**Patentansprüche für folgende Vertragsstaaten : AT, ES, GR**

**1.** Verfahren zur Herstellung eines α-Fluoracrylsäurephenylesters der Formel (1)

(1) 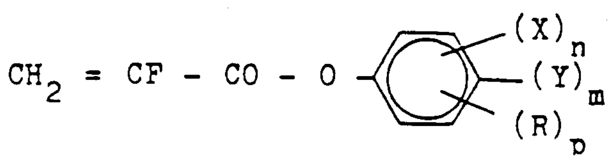

in der R eine Haloalkylgruppe mit 1 bis 8 Kohlenstoffatomen bedeutet und p Null, 1 , 2 oder 3 ist, X ein Halogenatom bedeutet und n Null oder eine ganze Zahl von 1 bis 5 ist (wobei X verschiedene Halogenatome bedeuten kann, wenn n größer als 1 ist), Y ein Wasserstoffatom, eine Cyanogruppe, den Rest X oder den Rest R bedeutet und m Null oder 1 ist, wobei die Summe n + m + p 2 bis 5 beträgt, dadurch gekennzeichnet, daß in einem ersten Verfahrensschritt α-Fluormalonsäure-dimethylester mit Formaldehyd umgesetzt wird, dann in einem zweiten Verfahrensschritt der erhaltene hydroxymethylierte α-Fluormalonsäure-dimethylester hydrolysiert, decarboxyliert und dehydratisiert wird und anschließend in einem dritten Verfahrensschritt die erhaltene α-Fluoracrylsäure (gegebenenfalls in Form eines Säurehalogenids) mit einem Phenol der Formel (2)

(2) 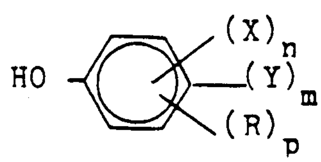

in der X, Y, R, n, m und p die bei Formel (1) angegebene Bedeutung haben (gegebenenfalls in Form eines Alkaliphenolats) verestert wird.

**2.** Verfahren nach Anspruch 1, dadurch gekennzeichnet, daß der erste Verfahrensschritt bei einer Temperatur von 5 bis 40° C, der zweite Verfahrensschritt bei einer Temperatur von 90 bis 110° C und der dritte Verfahrensschritt bei einer Temperatur von -10 bis 50° C durchgeführt werden.

**3.** Verwendung des nach Anspruch 1 hergestellten α-Fluoracrylsäurephenylesters der Formel (1) als Ausgangsmaterial zur Herstellung eines fluorhaltigen Polymers.

## Claims

### Claims for the following Contracting States : BE, CH, DE, FR, GB, IT, LI, LU, NL, SE

**1.** A phenyl α-fluoroacrylate of the formula (1)

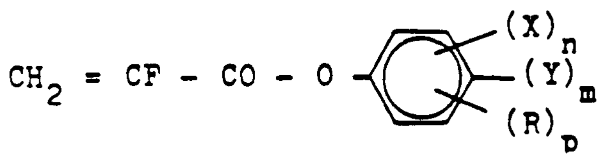

in which R denotes a haloalkyl group having 1 to 8 carbon atoms and p is zero, 1, 2 or 3, X denotes a halogen atom and n is zero or an integer from 1 to 5 (it being possible for X to denote different halogen atoms if n is greater than 1), Y denotes a hydrogen atom, a cyano group, the radical X or the radical R and m is zero or 1, the sum of n + m + p being 2 to 5.

**2.** A process for the preparation of a phenyl α-fluoroacrylate, which comprises reacting dimethyl α-fluoromalonate with formaldehyde in a first process stage, then hydrolysing, decarboxylating and dehydrating the resulting hydroxymethylated dimethyl α-fluoromalonate in a second process stage, and subsequently esterifying, in a third process stage, the resulting α-fluoroacrylic acid (if appropriate in the form of an acid halide) with a phenol of the formula (2)

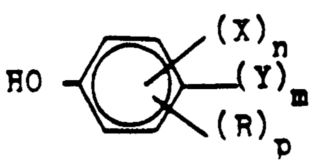

(2)

in which X, Y, R, n, m and p have the meaning indicated in formula (1) (if appropriate in the form of an alkali metal phenolate).

**3.** The process as claimed in claim 2, wherein the first process stage is carried out at a temperature from 5 to 40 °C, the second process stage is carried out at a temperature from 90 to 110 °C, and the third process stage is carried out at a temperature from -10 to 50 °C.

**4.** The use of the phenyl α-fluoroacrylate of the formula (1) as a starting material for the preparation of a fluorine-containing polymer.

### Claims for the following Contracting States : AT, ES, GR

**1.** A process for the preparation of a phenyl α-fluoroacrylate of the formula (1)

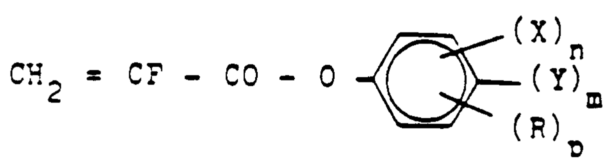

(1)

in which R denotes a haloalkyl group having 1 to 8 carbon atoms and p is zero, 1, 2 or 3, X denotes a halogen atom and n is zero or an integer from 1 to 5 (it being possible for X to denote different halogen

atoms if n is greater than 1), Y denotes a hydrogen atom, a cyano group, the radical X or the radical R and m is zero or 1, the sum of n + m + p being 2 to 5, which comprises reacting dimethyl α-fluoromalonate with formaldehyde in a first process stage, then hydrolyzing, decarboxylating and dehydrating the resulting hydroxymethylated dimethyl α-fluoromalonate in a second process stage, and subsequently esterifying, in a third process stage, the resulting α-fluoroacrylic acid (if appropriate in the form of an acid halide) with a phenol of the formula (2)

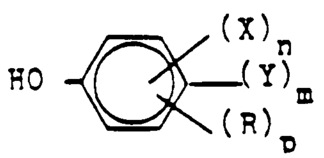

(2)

in which X, Y, R, n, m and p have the meaning indicated in formula (1) (if appropriate in the form of an alkali metal phenolate).

2. The process as claimed in claim 1, wherein the first process stage is carried out at a temperature from 5 to 40 °C, the second process stage is carried out at a temperature from 90 to 110 °C, and the third process stage is carried out at a temperature from -10 to 50 °C.

3. The use of the phenyl α-fluoroacrylate of the formula (1) prepared as claimed in claim 1 as a starting material for the preparation of a fluorine-containing polymer.

**Revendications**
**Revendications pour les Etats contractants suivants : BE, CH, DE, FR, GR, IT, LI, LU, NL, SE**

1. Esters phényliques de l'acide α-fluoracrylique qui répondent à la formule 1 :

(1) $CH_2 = CF - CO - O$ 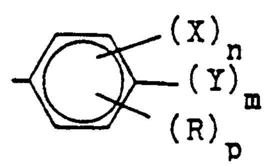

dans laquelle R représente un radical halogéno-alkyle contenant de 1 à 8 atomes de carbone, p est égal à 0, à 1, à 2 ou à 3, X représente un atome d'halogène, n désigne un nombre entier de 0 à 5 (les X représentant des atomes d'halogènes différents lorsque n est supérieur à 1), Y représente un atome d'hydrogène, un radical cyano, le radical X ou le radical R, et m est égal à 0 ou à 1, la somme (n + m + p) étant égale à un nombre de 2 à 5.

2. Procédé pour préparer un ester phénylique de l'acide α-fluoracrylique, procédé caractérisé en ce que, dans une première étape opératoire, on fait réagir l'α-fluoromalonate de diméthyle avec le formaldéhyde, puis, dans une deuxième étape opératoire, on hydrolyse l'α-fluoromalonate de diméthyle hvdroxyméthylé obtenu, on le décarboxyle et on le déshydrate, après quoi, dans une troisième étape opératoire, on estérifie l'acide α-fluoracrylique obtenu (éventuellement sous la forme d'un halogénure d'acide) avec un phénol répondant à la formule 2:

(2) 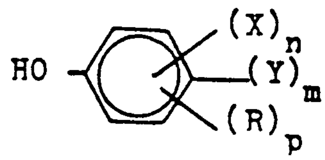

(dans laquelle X, Y, R, n, m et p ont les significations indiquées à propos de la formule 1), éventuellement sous la forme d'un phénolate de métal alcalin.

3. Procédé selon la revendication 2 caractérisé en ce que la première étape opératoire est exécutée à une température de 5 à 40 ° C, la deuxième à une température de 90 à 110 ° C et la troisième à une température de -10 à 50 ° C.

4. Application de l'α-fluoracrylate de phényle de formule 1 comme corps de départ pour la préparation d'un polymère fluoré.

**Revendications pour les Etats contractants suivants : AT, ES, GR**

1. Procédé pour préparer un α-fluoracrylate de phényle répondant à la formule 1 :

(1) 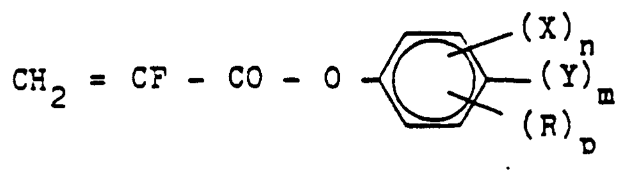

dans laquelle R représente un radical halogéno-alkyle contenant de 1 à 8 atomes de carbone, p est égal à 0, à 1, à 2 ou à 3, X représente un atome d'halogène, n désigne un nombre entier de 0 à 5 (les X représentant des atomes d'halogènes différents lorsque n est supérieur à 1), Y représente un atome d'hydrogène, un radical cyano, le radical X ou le radical R, et m est égal à 0 ou à 1, la somme (n + m + p) étant égale à un nombre de 2 à 5,
procédé caractérisé en ce que, dans une première étape opératoire, on fait réagir l'α-fluoromalonate de diméthyle avec le formaldéhyde, puis, dans une seconde étape opératoire, on hydrolyse l'α-fluoromalonate de diméthyle hydroxyméthylé obtenu, on le décarboxyle et on le déshydrate, après quoi, dans une troisième étape opératoire, on estérifie l'acide α-fluoracrylique obtenu (éventuellement sous la forme d'un halogénure d'acide) avec un phénol répondant à la formule 2 :

(2) 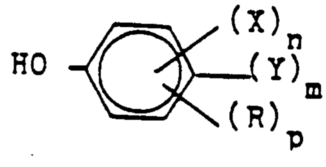

(dans laquelle X, Y, R, n, m et p ont les significations indiquées à propos de la formule 1), éventuellement sous la forme d'un phénolate de métal alcalin.

2. Procédé selon la revendication 1 caractérisé en ce que la première étape opératoire est exécutée à une température de 5 à 40 ° C, la deuxième à une température de 90 à 110 ° C et la troisième à une température de -10 à 50 ° C.

**3.** Application de l'α-fluoracrylate de phényle de formule 1 qui a été préparé selon la revendicaiton 1 comme corps de départ pour la préparation d'un polymère fluoré.